# EUROPEAN PATENT APPLICATION

(11) **EP 4 671 800 A2**
(43) Date of publication of application: **31.12.2025**
(21) Application number: 25184316.5
(22) Date of filing: 20.06.2025
(51) Int. Cl.: G01R 33/54, A61B 5/00, G01R 33/56

(54) **MAGNETIC RESONANCE IMAGING APPARATUS, POSITIONING ASSISTANCE METHOD, AND PROGRAM**

(30) Priority: 24.06.2024 JP 2024101252
(71) Applicant: FUJIFILM Corporation, Tokyo 106-8620 (JP)
(72) Inventor: HATORI, Shohei, Tokyo, 1068620 (JP); TAKAHASHI, Shinji, Tokyo, 1068620 (JP)
(74) Representative: Dehns Germany Partnerschaft mbB

(57) **Abstract**

A processor (202) of a magnetic resonance imaging apparatus (10) acquires a three-dimensional image including a heart of a subject (130) through scanogram imaging executed prior to main imaging, detects a landmark of the heart from the three-dimensional image, sets a position of a cross-section (CS) based on the landmark, generates a cross-sectional image (F9A, F10A) appearing on the cross-section from the three-dimensional image, sets a horizontal direction or a vertical direction of the cross-sectional image (F9A, F10A) such that the horizontal direction or the vertical direction of the cross-sectional image (F9A, F10A) is parallel to a straight line obtained by projecting one axis selected from among three axes that form a three-dimensional orthogonal coordinate system of a real space onto the cross-sectional image (F9A, F10A), and stores the cross-sectional image (F9A, F10A) in a memory (204).

## Description

### BACKGROUND OF THE INVENTION

### 1. Field of the Invention

The present disclosure relates to a magnetic resonance imaging (MRI) apparatus, a positioning assistance method, and a program, and more particularly, to a cross-sectional image generation technique and a positioning assistance technique for assisting in setting an imaging position in a cardiac MRI examination.

### 2. Description of the Related Art

In the cardiac MRI examination, imaging of a reference cross-section specified based on anatomical features of a heart is performed. Useful reference cross-sectional images for diagnosis include, for example, a left ventricular short-axis image, a horizontal long-axis image, a vertical long-axis image, a four-chamber long-axis image, a two-chamber long-axis image, a three-chamber long-axis image, and the like, and for each examination, the imaging position of each cross-section is set in accordance with a subject. JP2012-110688A describes a technique for assisting in positioning each reference cross-section.

An MRI apparatus described in JP2012-110688A comprises a collection unit that collects a plurality of pieces of cross-sectional image data including a heart from a subject by using magnetic resonance; a reference cross-section information calculation unit that calculates spatial position information of a reference cross-section of the heart based on the plurality of pieces of cross-sectional image data; a positioning unit that displays a reference cross-sectional image of the heart calculated from the plurality of pieces of cross-sectional image data based on the position information of the reference cross-section on a display device and that performs positioning of an imaging site for imaging through the displayed reference cross-sectional image of the heart; and an imaging unit that images the imaging site set by the positioning.

### SUMMARY OF THE INVENTION

In the MRI apparatus described in JP2012-110688A, multi-slice axial cross-sectional image data covering the entire heart of the subject is acquired through preliminary imaging before main imaging, and three-dimensional (3D) volume image data of the heart is generated through isotropic processing. Then, from this 3D volume image data, positions such as an apex of the heart, a mitral valve, a long axis, and a left ventricular center are detected using a pattern matching method, and a cross-section is set based on these landmarks. Based on the cross-section set in this manner, a user, such as a technician, sets an imaging field of view (FOV) and performs the main imaging.

The preliminary imaging corresponds to scanogram imaging in the present specification. The multi-slice axial cross-sectional image data or the 3D volume image data corresponds to a three-dimensional image in the present specification.

In the related art, among a plurality of reference cross-sections used in the cardiac MRI examination, particularly for the cross-sections shown in Fig. (B), (E), (F), (H), and (I) of Fig. 6 of JP2012-110688A (a left ventricular short-axis image, a horizontal long-axis image, a four-chamber long-axis image, a two-chamber long-axis image, and a three-chamber long-axis image), the imaging cross-sections are set with respect to the long axis of the heart. Therefore, angles of these cross-sections are tilted with respect to three orthogonal axes of a body axis (see Fig. 6 of JP2012-110688A).

Since a morphology and an orientation of the heart in a body vary among individuals and differ between subjects, structures outside the heart within the cross-section set based on landmarks may be orientated in various directions depending on the individual. Consequently, for example, structures outside the imaging field of view may appear inside the imaging field of view, causing the occurrence of aliasing artifacts. As a result, the user needs to perform tasks such as adjusting the angle of display of the cross-sectional image or changing setting of the imaging field of view for each subject.

The present disclosure has been made in view of such circumstances, and an object of the present disclosure is to provide a magnetic resonance imaging apparatus, a positioning assistance method, and a program that ensure that structures within a cross-section are output at the same angle, regardless of a subject, in a case of generating a cross-sectional image of a heart from a three-dimensional image obtained by scanogram imaging.

According to a first aspect of the present disclosure, there is provided a magnetic resonance imaging apparatus that generates an image of a subject based on nuclear magnetic resonance, the apparatus comprising: a processor; and a memory, in which the processor is configured to: acquire a three-dimensional image including a heart of the subject through scanogram imaging executed prior to main imaging; detect a landmark of the heart from the three-dimensional image; set a position of a cross-section based on the landmark; generate a cross-sectional image appearing on the cross-section from the three-dimensional image; set a horizontal direction or a vertical direction of the cross-sectional image such that the horizontal direction or the vertical direction of the cross-sectional image is parallel to a straight line obtained by projecting one axis selected from among three axes that form a three-dimensional orthogonal coordinate system of a real space onto the cross-sectional image; and store the cross-sectional image in the memory.

The scanogram imaging is synonymous with preliminary imaging, positioning imaging, or pre-scan. The three-dimensional image obtained by the scanogram imaging may be, for example, a multi-slice image consisting of a plurality of slice images such as axial cross-sections, or may be an isotropic three-dimensional volume image obtained by performing isotropic processing on the multi-slice image. The landmark may be, for example, an anatomical feature site such as an apex of the heart, a mitral valve, a tricuspid valve, or a left ventricular outflow tract.

The three axes that form the three-dimensional orthogonal coordinate system of the real space may be, for example, an X-axis, a Y-axis, and a Z-axis as three axes of the magnetic resonance imaging apparatus, which are coordinate axes uniquely defined in the apparatus. The three axes of the apparatus can form a coordinate system of a three-dimensional image obtained in a case where the subject is imaged by the scanogram imaging. Each axis direction of a head-foot direction (HF direction), a right-left direction (RL direction), and an anterior-posterior direction (AP direction) of the subject placed in the imaging space can correspond to directions of three axes in a case where the three-dimensional image is captured. One axis selected from among the three axes may be defined to correspond to a target cross-section.

According to the first aspect, a position of the cross-section is determined based on the landmark detected from the three-dimensional image obtained by the scanogram imaging, and the cross-sectional image in a case where the three-dimensional image is cut at the cross-section position is generated. In defining the horizontal direction and the vertical direction (that is, right-left and up-down directions of the image) of the cross-sectional image, the processor refers to a straight line (a straight line projected onto the cross-sectional image) that appears on the cross-sectional image in a case where one axis selected from among the three axes of the real space is projected onto the cross-section, and sets the horizontal direction or the vertical direction of the cross-sectional image such that the straight line projected onto the cross-sectional image and the horizontal direction or the vertical direction of the cross-sectional image are parallel to each other.

The horizontal direction of the cross-sectional image means a lateral direction (right-left direction) on the image, and the vertical direction of the cross-sectional image means a longitudinal direction (up-down direction) perpendicular to the horizontal direction within an image plane. In a case where one direction of the horizontal direction or the vertical direction of the cross-sectional image is determined, the other direction perpendicular to the direction is also defined on the same image. Therefore, setting the horizontal direction or the vertical direction of the cross-sectional image includes setting the horizontal direction and the vertical direction of the cross-sectional image. In this manner, the cross-sectional image in which the horizontal direction or the vertical direction of the cross-sectional image is set in accordance with the directions of the axes of the real space is stored in the memory. According to the first aspect, a cross-sectional image can be obtained in which structures within the cross-section are output at an angle facing the same direction, regardless of the subject.

The term "horizontal" in the present specification may include a concept of being substantially horizontal, which can be regarded as having a range substantially equivalent to being horizontal in a technical sense. The term "vertical" may include a concept of being substantially vertical, which can be regarded as having a range substantially equivalent to being vertical in a technical sense. In addition, the term "parallel" may include the concept of being substantially parallel, which can be regarded as having a range substantially equivalent to being parallel in a technical sense.

According to a second aspect, in the magnetic resonance imaging apparatus according to the first aspect, the processor may be configured to: rotate the cross-sectional image at an angle at which the horizontal direction or the vertical direction of the cross-sectional image is parallel to the straight line projected onto the cross-sectional image; and store the rotated cross-sectional image in the memory.

Setting the angle of rotation of the cross-sectional image is understood as one aspect of setting the horizontal direction or the vertical direction of the cross-sectional image.

According to a third aspect, in the magnetic resonance imaging apparatus according to the first or second aspect, a display device may be further provided, and the processor may be configured to: display the cross-sectional image on the display device.

The cross-sectional image stored in the memory can be output to the display device. The cross-sectional image displayed on the display device is displayed on a screen, with the horizontal direction and the vertical direction of the cross-sectional image matching the horizontal (lateral) direction and the vertical (longitudinal) direction of the screen of the display device. As a result, the cross-sectional image can be displayed with the orientations of the structures within the cross-section aligned, regardless of the subject.

According to a fourth aspect, in the magnetic resonance imaging apparatus according to any one of the first to third aspects, the processor may be configured to: generate a display image in which a frame indicating a region of an imaging field of view is superimposed on the cross-sectional image.

The imaging field of view is for setting an imaging range of the main imaging, and it is preferable to display the frame indicating the region of the imaging field of view (imaging field of view frame) together with the cross-sectional image during the positioning.

According to a fifth aspect, in the magnetic resonance imaging apparatus according to the fourth aspect, the frame indicating the region of the imaging field of view displayed on the cross-sectional image may be a quadrangular frame surrounded by one set of opposing sides parallel to the horizontal direction of the cross-sectional image and one set of opposing sides parallel to the vertical direction of the cross-sectional image.

For example, the frame indicating the region of the automatically set imaging field of view can be displayed as an upright quadrangular frame with respect to the horizontal direction and the vertical direction of the cross-sectional image.

According to a sixth aspect, in the magnetic resonance imaging apparatus according to any one of the first to fifth aspects, the processor may be configured to: detect the landmark using a first artificial intelligence model that has been trained through machine learning.

According to a seventh aspect, in the magnetic resonance imaging apparatus according to any one of the first to sixth aspects, the processor may be configured to: extract a body surface from the three-dimensional image; set a body axis center based on the extracted body surface; set a center of a structure within the cross-section based on the body axis center; and set the imaging field of view for the cross-sectional image such that a center of the imaging field of view matches the center of the structure within the cross-section.

By matching the center of the structure within the cross-section with the center of the imaging field of view, the structure outside the imaging field of view, which is a factor of artifacts, can be minimized.

According to an eighth aspect, in the magnetic resonance imaging apparatus according to the seventh aspect, the processor may be configured to: extract the body surface using a second artificial intelligence model that has been trained through machine learning.

According to a ninth aspect, in the magnetic resonance imaging apparatus according to any one of the first to eighth aspects, a configuration may be employed in which the horizontal direction or the vertical direction of the cross-sectional image is a phase-encoding direction.

According to the ninth aspect, it is possible to suppress the structure outside the imaging field of view in the phase-encoding direction in which artifacts are likely to occur.

According to a tenth aspect, in the magnetic resonance imaging apparatus according to any one of the first to ninth aspects, a configuration may be employed in which the cross-section includes a cross-section from which at least one of a left ventricular short-axis image, a horizontal long-axis image, a four-chamber long-axis image, a two-chamber long-axis image, or a three-chamber long-axis image is obtained.

The processor may generate a cross-sectional image for one cross-section of a plurality of reference cross-sections used in a cardiac MRI examination or may generate a cross-sectional image for each of two or more cross-sections of the plurality of reference cross-sections. It is preferable that the processor is configured to generate cross-sectional images for all of the plurality of reference cross-sections.

According to an eleventh aspect, in the magnetic resonance imaging apparatus according to any one of the first to tenth aspects, the cross-section may be a reference cross-section from which a left ventricular short-axis image is obtained, and the processor may be configured to: in a case where the cross-section includes a component in an AP direction which is an anterior-posterior direction of the subject, rotate the cross-sectional image at an angle at which the horizontal direction of the cross-sectional image is parallel to a straight line obtained by projecting an AP axis as the one axis onto the cross-sectional image.

According to a twelfth aspect, in the magnetic resonance imaging apparatus according to the eleventh aspect, the processor may be configured to: in a case where the cross-section does not include the component in the AP direction, set the horizontal direction and the vertical direction of the cross-sectional image to be the same as a horizontal direction and a vertical direction of a coronal cross-sectional image.

According to a thirteenth aspect, in the magnetic resonance imaging apparatus according to any one of the first to tenth aspects, the cross-section may be a reference cross-section from which a horizontal long-axis image is obtained, and the processor may be configured to: in a case where the cross-section includes a component in an RL direction which is a right-left direction of the subject, rotate the cross-sectional image at an angle at which the horizontal direction of the cross-sectional image is parallel to a straight line obtained by projecting an RL axis as the one axis onto the cross-sectional image.

According to a fourteenth aspect, in the magnetic resonance imaging apparatus according to the thirteenth aspect, the processor may be configured to: in a case where the cross-section does not include the component in the RL direction, set the horizontal direction and the vertical direction of the cross-sectional image to be the same as a horizontal direction and a vertical direction of a sagittal cross-sectional image.

According to a fifteenth aspect, in the magnetic resonance imaging apparatus according to any one of the first to tenth aspects, the cross-section may be a reference cross-section from which a four-chamber long-axis image is obtained, and the processor may be configured to: in a case where the cross-section includes a component in an RL direction which is a right-left direction of the subject, rotate the cross-sectional image at an angle at which the horizontal direction of the cross-sectional image is parallel to a straight line obtained by projecting an RL axis as the one axis onto the cross-sectional image.

According to a sixteenth aspect, in the magnetic resonance imaging apparatus according to the fifteenth aspect, the processor may be configured to: in a case where the cross-section does not include the component in the RL direction, set the horizontal direction and the vertical direction of the cross-sectional image to be the same as a horizontal direction and a vertical direction of a sagittal cross-sectional image.

According to a seventeenth aspect, in the magnetic resonance imaging apparatus according to any one of the first to tenth aspects, the cross-section may be a reference cross-section from which a two-chamber long-axis image is obtained, and the processor may be configured to: in a case where the cross-section includes a component in an HF direction which is a head-foot direction of the subject, rotate the cross-sectional image at an angle at which the vertical direction of the cross-sectional image is parallel to a straight line obtained by projecting an HF axis as the one axis onto the cross-sectional image.

According to an eighteenth aspect, in the magnetic resonance imaging apparatus according to the seventeenth aspect, the processor may be configured to: in a case where the cross-section does not include the component in the HF direction, set the horizontal direction and the vertical direction of the cross-sectional image to be the same as a horizontal direction and a vertical direction of an axial cross-sectional image.

According to a nineteenth aspect, in the magnetic resonance imaging apparatus according to any one of the first to tenth aspects, the cross-section may be a reference cross-section from which a three-chamber long-axis image is obtained, and the processor may be configured to: in a case where the cross-section includes a component in an AP direction which is an anterior-posterior direction of the subject, rotate the cross-sectional image at an angle at which the vertical direction of the cross-sectional image is parallel to a straight line obtained by projecting an AP axis as the one axis onto the cross-sectional image.

According to a twentieth aspect, in the magnetic resonance imaging apparatus according to the nineteenth aspect, the processor may be configured to: in a case where the cross-section does not include the component in the AP direction, set the horizontal direction and the vertical direction of the cross-sectional image to be the same as a horizontal direction and a vertical direction of a coronal cross-sectional image.

According to a twenty-first aspect, there is provided a positioning assistance method of assisting in setting an imaging position by a magnetic resonance imaging apparatus, the method comprising: causing a processor to execute: a step of acquiring a three-dimensional image including a heart of a subject through scanogram imaging executed prior to main imaging; a step of detecting a landmark of the heart from the three-dimensional image; a step of setting a position of a cross-section based on the landmark; a step of generating a cross-sectional image appearing on the cross-section from the three-dimensional image; a step of setting a horizontal direction or a vertical direction of the cross-sectional image such that the horizontal direction or the vertical direction of the cross-sectional image is parallel to a straight line obtained by projecting one axis selected from among three axes that form a three-dimensional orthogonal coordinate system of a real space onto the cross-sectional image; and a step of storing the cross-sectional image in a memory.

The processor may automatically execute the processing of each step in accordance with a program or may accept an input of an instruction from a user for a part or all of the steps and execute the processing according to the accepted instruction.

For the positioning assistance method according to the twenty-first aspect, a configuration can be employed in which the same specific aspect as the magnetic resonance imaging apparatus according to any one of the second to twentieth aspects is included.

According to a twenty-second aspect, there is provided a program for causing a computer to execute the positioning assistance method according to the twenty-first aspect.

For the program according to the twenty-second aspect, a configuration can be employed in which the same specific aspect as the magnetic resonance imaging apparatus according to any one of the second to twentieth aspects is included.

The present disclosure also includes a non-transitory and tangible computer-readable storage medium in which the program according to the twenty-second aspect is stored.

According to the present disclosure, it is possible to generate a cross-sectional image in which structures within a cross-section are output at the same angle, regardless of a subject, in a case of generating a cross-sectional image of a heart of the subject from a three-dimensional image obtained by scanogram imaging.

### BRIEF DESCRIPTION OF THE DRAWINGS

Fig. 1 is a perspective view showing an external appearance of an exemplary magnetic resonance imaging (MRI) apparatus.
Fig. 2 is a diagram showing a schematic configuration of an inside of the MRI apparatus.
Fig. 3 is a block diagram showing an example of a hardware configuration of an information processing apparatus used in the MRI apparatus.
Fig. 4 is an explanatory diagram of aliasing artifacts that occur due to structures outside an imaging field of view.
Fig. 5 is an example of a cross-sectional image in a case where a structure, which is a factor of artifacts, exists outside the imaging field of view.
Fig. 6 is a flowchart showing an example of a case where an imaging position of a left ventricular short-axis image in the MRI apparatus is set.
Fig. 7 is an explanatory diagram showing an example of cross-section cutout processing in step S3 of Fig. 6.
Fig. 8 is an explanatory diagram showing an example of a method of setting a center of the imaging field of view of the left ventricular short-axis image applied from step S4 to step S6 of Fig. 6.
Fig. 9 is an explanatory diagram showing a state of the cross-sectional image before rotation, which has been cut out from a 3D scanogram image.
Fig. 10 is an example of the cross-sectional image after rotation, which is generated in step S7 of Fig. 6.
Fig. 11 is an explanatory diagram showing an example of setting a horizontal direction of the left ventricular short-axis image in a case where a cross-section cut out from the 3D scanogram image includes a component in an AP direction, and shows a straight line of each axis projected onto the cross-section.
Fig. 12 is an explanatory diagram showing an example of setting the horizontal direction of the left ventricular short-axis image in a case where a cross-section cut out from the 3D scanogram image does not include the component in the AP direction.
Fig. 13 is an explanatory diagram showing an example of a user interface in a case of performing cross-section display of the left ventricular short-axis image.
Fig. 14 is a block diagram showing a functional configuration of the information processing apparatus used in the MRI apparatus.

### DESCRIPTION OF THE PREFERRED EMBODIMENTS

Preferred embodiments of the present invention will be described in detail below with reference to the accompanying drawings. In the following description and the accompanying drawings, components having the identical functional configuration will be denoted by the identical reference numerals, and duplicated descriptions will not be repeated.

### Overview of Magnetic Resonance Imaging (MRI) Apparatus

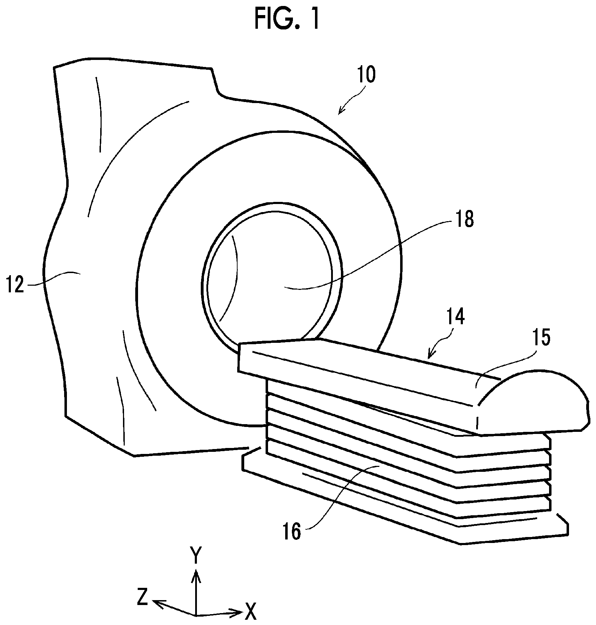

Fig. 1 is a perspective view showing an external appearance of an exemplary MRI apparatus 10. The MRI apparatus 10 comprises a gantry 12, which is an imaging apparatus main body, and a patient table 14. The gantry 12 includes a cylindrical imaging space 18 called a bore. In the gantry 12, a static magnetic field generating magnet, a gradient magnetic field coil, and various other coils for generating a magnetic field in the imaging space 18 are disposed.

The patient table 14 comprises a top plate 15 and a leg portion 16 that supports the top plate 15, and is disposed on a front surface side of the gantry 12. The top plate 15 can be advanced into the imaging space 18 and retracted from the imaging space 18 by a drive mechanism (not shown) provided in the leg portion 16. The patient table 14 may be fixed to the gantry 12 or may be a movable patient table (dockable patient table) that is attachable to and detachable from the gantry 12.

In the MRI apparatus 10, three axes of the apparatus, which are three-dimensional orthogonal coordinate axes of a real space including the imaging space 18, are defined. The direction of each axis of the three axes of the apparatus is uniquely determined from the gantry 12 and the patient table 14. A Z direction in Fig. 1 is a static magnetic field direction, a Y direction is a vertical direction, and an X direction is a direction orthogonal to the Y direction and the Z direction.

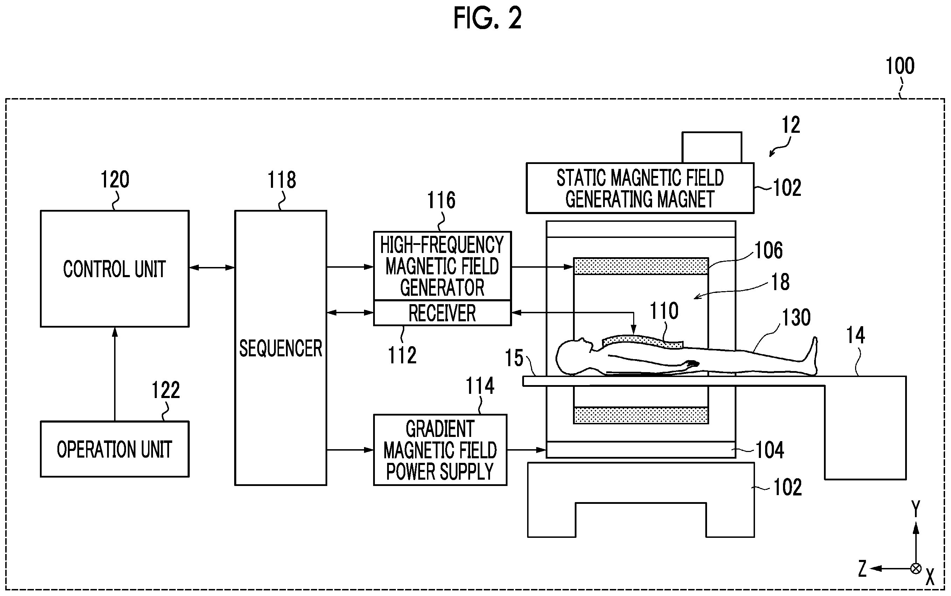

Fig. 2 is a diagram showing a schematic configuration of an inside of the MRI apparatus 10. The MRI apparatus 10 comprises a static magnetic field generating magnet 102, a gradient magnetic field coil 104, a radio frequency (RF) transmit coil 106, a receive coil 110, a receiver 112, a gradient magnetic field power supply 114, a high-frequency magnetic field generator 116, a sequencer 118, a control unit 120, and an operation unit 122.

The static magnetic field generating magnet 102 generates a uniform static magnetic field in the imaging space 18. The static magnetic field generating magnet 102 includes a static magnetic field generating source of a permanent magnet type, a normal conducting type, or a superconducting type. The gradient magnetic field coil 104 generates a gradient magnetic field in the imaging space 18. The gradient magnetic field coil 104 is composed of gradient magnetic field coils in three-axis directions of X, Y, and Z in a real space coordinate system (stationary coordinate system). Each gradient magnetic field coil is connected to a gradient magnetic field power supply 114 and is supplied with a current. As a result, a gradient magnetic field is generated in the three-axis directions of X, Y, and Z.

A subject 130 is placed on the top plate 15 of the patient table 14, and the receive coil 110 corresponding to an examination site is attached to the subject 130. By moving the top plate 15 on which the subject 130 is placed into the imaging space 18, the examination site of the subject 130 is positioned at a static magnetic field center in the imaging space 18.

The RF transmit coil 106 is a coil that irradiates the subject 130 with a high-frequency magnetic field pulse (RF pulse). The RF transmit coil 106 is connected to the high-frequency magnetic field generator 116 and is supplied with a high-frequency pulse current from the high-frequency magnetic field generator 116.

The sequencer 118 transmits commands to the high-frequency magnetic field generator 116 and the gradient magnetic field power supply 114 in accordance with an imaging pulse sequence. As a result, signals amplified appropriately are transmitted to the RF transmit coil 106 and the gradient magnetic field coil 104, respectively.

The high-frequency magnetic field generator 116 is driven in accordance with a command from the sequencer 118, modulates the amplitude of a high-frequency pulse, and supplies the amplified high-frequency pulse current to the RF transmit coil 106. The RF transmit coil 106 applies a pulsed high-frequency magnetic field (RF pulse) to the subject 130 in accordance with a signal from the high-frequency magnetic field generator 116. Preferably, the sequencer 118 transmits a command to the receive coil 110 in accordance with the above-described imaging sequence and turns off the receive coil 110 during RF pulse application.

The application of the high-frequency magnetic field induces a nuclear magnetic resonance (NMR) phenomenon in spins of atoms constituting a biological tissue of the subject 130. The receive coil 110 is a multi-channel RF coil unit including a plurality of coil elements for receiving nuclear magnetic resonance signals (NMR signals) generated in the subject 130.

The NMR signal generated from the subject 130 is detected by the receive coil 110, is amplified by a preamplifier (not shown) in the receive coil 110, and is transmitted to the receiver 112. In the receiver 112, the amplified NMR signal is subjected to analog-to-digital (AD) conversion and necessary signal processing to generate data. The generated data is transmitted to the control unit 120. This data is also referred to as a received signal or measurement data.

A reception-side cable (not shown) that outputs the NMR signal received by the receive coil 110 is connected to the receive coil 110. A reception-side connector (not shown) is connected to an end portion of the reception-side cable. The reception-side connector is connected to a patient table-side connector of a patient table-side cable (not shown). The patient table-side cable is connected to the control unit 120. Consequently, the receive coil 110, the control unit 120, and the sequencer 118 are communicably connected to each other. The connection between the receive coil 110, and the control unit 120 and the sequencer 118 is not limited to wired connection, such as through a cable, and can also be established wirelessly. In this case, the receive coil 110 further includes at least an AD conversion module and a wireless communication module.

The sequencer 118 controls each unit to operate at a pre-programmed timing and intensity. Among programs, a program that particularly describes the timing and intensity of the RF pulse, the gradient magnetic field, and signal reception is called a pulse sequence. Various pulse sequences are known depending on the purpose, but detailed descriptions thereof will be omitted here.

The control unit 120 accepts various instruction inputs from the operation unit 122 and integrally controls each unit of the MRI apparatus 10 via the sequencer 118. Additionally, the control unit 120 performs processing such as inverse Fourier transforming the received signal in a spatial frequency domain received from the receiver 112 to convert the received signal into an image in the real space, thereby generating an MRI image.

The operation unit 122 includes an input device such as a mouse and a keyboard, and a display device such as a liquid crystal display. The operation unit 122 functions as a user interface (UI) for a user, such as a technician, to start and stop (pause) the MRI apparatus 10, select a pulse sequence, input imaging conditions or processing conditions, and the like.

The sequencer 118 and the control unit 120 can be configured using a computer. In addition, processing functions of the sequencer 118 and the control unit 120 may be implemented by a computer system including a plurality of computers.

### Example of Hardware Configuration of Information Processing Apparatus

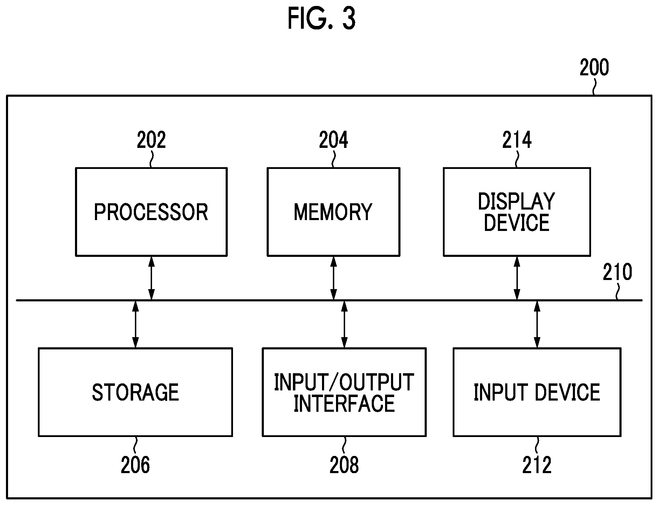

Fig. 3 is a block diagram showing an example of a hardware configuration of an information processing apparatus 200 used in the MRI apparatus 10. The information processing apparatus 200 may be a personal computer, a workstation, or a server computer. The information processing apparatus 200 can function as the control unit 120 and the operation unit 122 (see Fig. 2). The information processing apparatus 200 may function as the sequencer 118.

The information processing apparatus 200 comprises a processor 202, a memory 204 that is a main storage device, a storage 206 that is an auxiliary storage device, an input/output interface 208, and a bus 210.

The processor 202 includes a central processing unit (CPU). The processor 202 may include a graphics processing unit (GPU). Additionally, the information processing apparatus 200 may include one or a plurality of processors of a digital signal processor (DSP), an application-specific integrated circuit (ASIC), a programmable logic device (PLD), and the like.

The processor 202 is connected to the memory 204, the storage 206, the input/output interface 208, an input device 212, and a display device 214 via the bus 210.

The memory 204 includes a random access memory (RAM). The memory 204 may include a read only memory (ROM). The storage 206 may be, for example, a hard disk drive (HDD), a solid state drive (SSD), or a combination of a plurality of such devices. Additionally, the storage 206 may include an external storage device, such as a removable medium.

The storage device including the memory 204 and the storage 206 stores programs, data, and the like for implementing various functions of the MRI apparatus 10. The processor 202 executes the programs stored in the memory 204 to implement various functions. The processor 202 integrally controls each unit of the information processing apparatus 200, various devices and units provided in the MRI apparatus 10, and the like and performs various kinds of processing.

The input/output interface 208 includes a communication interface that is connectable to telecommunication lines such as a local area network, a connection interface that is connectable to external devices, and the like. As the connection interface that is connectable to the external devices, for example, a Universal Serial Bus, a High-Definition Multimedia Interface (HDMI) (HDMI is a registered trademark), or the like can be applied.

The processor 202 communicates with various devices of the MRI apparatus 10 via the input/output interface 208, thereby transmitting and receiving necessary information.

The input device 212 includes, for example, a keyboard, a pointing device such as a mouse, a ten-keypad, various switch buttons, and the like. The input device 212 may include a voice input device. In addition, the input device 212 may be a touch panel-type input device that is configured integrally with a display screen of the display device 214.

The display device 214 is configured as, for example, a liquid crystal display, an organic electro-luminescence (OEL) display, a projector, or an appropriate combination thereof. The display device 214 displays various types of information, in addition to the image captured by the MRI apparatus 10. The display device 214 is used as a part of the UI in a case of accepting an input via the input device 212. The display device 214 is not limited to a single display device, and a form of a multi-display comprising a plurality of display devices is also possible.

### Overview of Procedure of MRI Imaging

A typical imaging procedure by the MRI apparatus 10 is generally as follows.

[Procedure 1] An operator, such as a technician, uses the input device 212, such as a mouse and a keyboard, to set subject information, an imaging site (target disease), an examination protocol (imaging type, order, and the like), and other information necessary for the examination. The subject information includes a name of the subject, a subject code, and the like. A part or all of these pieces of information may be manually input by the operator via the input device 212 or may be read from information stored in a recording medium or the like in advance. The operator finally confirms parameters that are automatically set or input, and manually sets the parameters using the input device 212 as necessary.

[Procedure 2] The subject 130 is placed on the top plate 15 of the patient table 14, and the receive coil 110 is attached to the subject 130.

[Procedure 3] The top plate 15 is moved to the imaging space 18 using a gantry operation panel (not shown), a foot switch, or the like, and an examination target site of the subject 130 is moved to a static magnetic field center position of the gantry 12.

[Procedure 4] Scanogram imaging for acquiring a positioning image is executed. In scanogram imaging, imaging of a plurality of slices is performed for one or more cross-sections of an axial cross-section, a coronal cross-section, and a sagittal cross-section. In the case of the cardiac MRI examination, imaging is performed in synchronization with an electrocardiogram waveform for an imaging range covering a three-dimensional region including the heart of the subject 130 in the scanogram imaging, thereby acquiring a 3D scanogram image. A slice thickness in the scanogram imaging may be set to a value larger than a slice thickness in the main imaging.

[Procedure 5] After scanogram imaging ends, the positioning image is reconstructed, and the image is displayed on the display device 214 of the operation unit 122. Although details will be described below, the MRI apparatus 10 according to the present embodiment implements an automatic positioning function of automating the setting of the imaging position, and a landmark of the heart is automatically detected from the 3D scanogram image, and the position of the reference cross-section is determined using the landmark. Then, the cross-sectional image cut out at the determined position of the reference cross-section is presented on the display device 214.

[Procedure 6] The operator sets the imaging position of the main imaging based on the cross-sectional image generated from the 3D scanogram image. In the MRI apparatus 10 according to the present embodiment, the imaging position including a slice position (cross-section position) to be measured in the main imaging is automatically calculated from the 3D scanogram image, and a recommended imaging position is presented to the operator. The operator confirms the presented slice position and the like and manually adjusts the imaging position using the input device 212 of the operation unit 122 as necessary. Additionally, the operator sets imaging parameters to be applied to the main imaging. A part or all of the imaging parameters may be input by the user, such as a technician, via the operation unit 122 or may be automatically set or input. The operator finally confirms the automatically set (automatically input) parameters and manually sets the parameters using the input device 212 of the operation unit 122 as necessary.

[Procedure 7] The main imaging is executed in accordance with the imaging parameters and the imaging position set in this manner.

[Procedure 8] After the main imaging, the image is reconstructed, and the image is displayed on the display device 214 of the operation unit 122.

[Procedure 9] The operator sets a window value to a value suitable for diagnosis for the displayed reconstructed image using the input device 212 of the operation unit 122 to obtain an image to be used for diagnosis.

[Procedure 10] After the imaging is completed, the top plate 15 is retracted from the imaging space 18, and the subject 130 is removed from the gantry 12. Then, the subject 130 is made to get off the patient table 14, and the MRI examination ends.

The MRI apparatus 10 according to the present embodiment implements means for solving issues of the related art, particularly concerning the output of the reconstructed image for positioning and the imaging position setting assistance function of presenting the recommended imaging position in the operations of Procedures 4 to 6 among Procedures 1 to 10 described above.

### Regarding Artifacts from Structures Outside Imaging Field of View

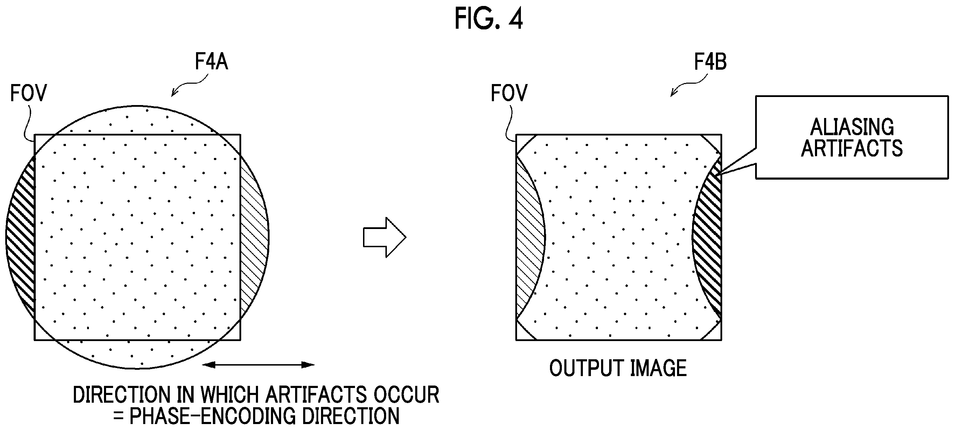

Here, a brief description of one of the technical issues, artifacts, will be provided. Fig. 4 is an explanatory diagram of aliasing artifacts that occur due to structures outside the imaging field of view. A circular region shown in a left diagram F4A of Fig. 4 schematically shows a structure inside the body as an imaging target, and the quadrangular region represents an imaging field of view FOV. In a case where a structure exists outside the imaging field of view FOV, aliasing artifacts may occur in an output image obtained by the main imaging, as shown in a right diagram F4B (right diagram F4B). Such artifacts significantly occur in a phase-encoding direction. In the example of Fig. 4, the horizontal direction (lateral direction) of the image is the phase-encoding direction.

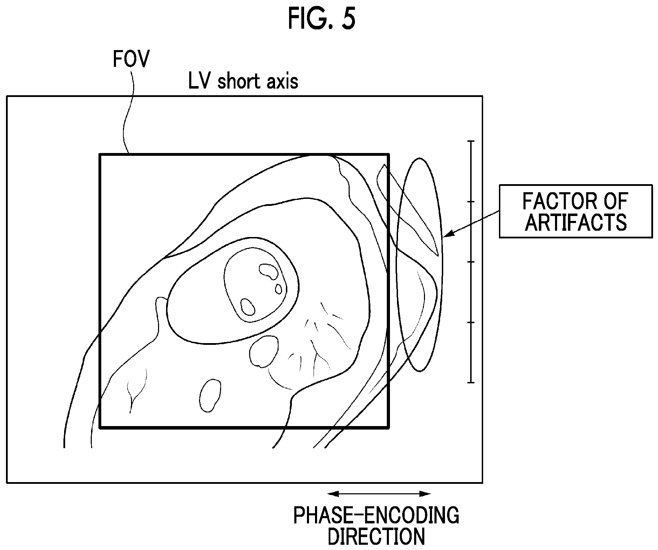

Fig. 5 is an example of a cross-sectional image in which a structure, which is a factor of artifacts, exists outside the imaging field of view FOV. Fig. 5 shows an example of the left ventricular short-axis image, and a quadrangular frame indicating a region of the imaging field of view FOV is displayed as an overlay on the image. The imaging field of view FOV shown in Fig. 5 is an example of the imaging field of view set by default and is set as a rectangular region surrounded by one set of opposing sides (an upper side and a lower side) parallel to the horizontal (lateral) direction of the cross-sectional image and one set of opposing sides (a left side and a right side) parallel to the vertical (longitudinal) direction of the cross-sectional image. Note that the term "rectangular" includes a square and a rectangle. The horizontal direction of the image in Fig. 5 is the phase-encoding direction.

In the case of the left ventricular short-axis image shown in Fig. 5, structures including the heart within the cross-section are displayed at an angle tilted with respect to the rectangular region of the imaging field of view FOV, and structures exist outside the imaging field of view FOV in the phase-encoding direction. Therefore, in the output image in a case where the main imaging is executed using the setting of the imaging field of view FOV shown in Fig. 5, aliasing artifacts may occur.

As described above, the morphology and the orientation of the heart vary depending on the individual, and the orientation of the body from which the reference cross-sectional image is obtained differs depending on the individual. Therefore, in a case where the cross-sectional image cut out from the cross-section determined based on the landmark detected from the 3D scanogram image is output to the display device 214, outputting the cross-sectional image without particularly considering a rotation angle (in-plane angle) of the cross-sectional image may cause differences in the orientation of the cross-sectional image displayed on the screen between subjects. As a result, as shown in Fig. 5, structures are more likely to exist outside the imaging field of view FOV in the phase-encoding direction, making artifacts more likely to occur.

In order to suppress the artifacts, the user needs to change the setting of the imaging field of view FOV or adjust the angle of the cross-sectional image with respect to the imaging field of view FOV.

Such an issue is not limited to the left ventricular short-axis image, and the same applies to other reference cross-sectional images such as a horizontal long-axis image, a four-chamber long-axis image, a two-chamber long-axis image, and a three-chamber long-axis image.

### Overview of Method of Generating Reference Cross-Sectional Image in MRI Apparatus 10

In the MRI apparatus 10 according to the present embodiment, the horizontal direction and the vertical direction of the cross-sectional image cut out from the 3D scanogram image are set such that the structures within the cross-section are aligned and displayed in the same orientation, regardless of the subject, for each cross-section of a plurality of reference cross-sections used in cardiac imaging. More specifically, the in-plane angle for rotating the cross-sectional image is set such that the structures outside the imaging field of view FOV in the phase-encoding direction, which cause the occurrence of artifacts, are minimized, and preferably, no structures exist outside the imaging field of view FOV in the phase-encoding direction, and the orientation of the cross-sectional image is adjusted through the rotation processing.

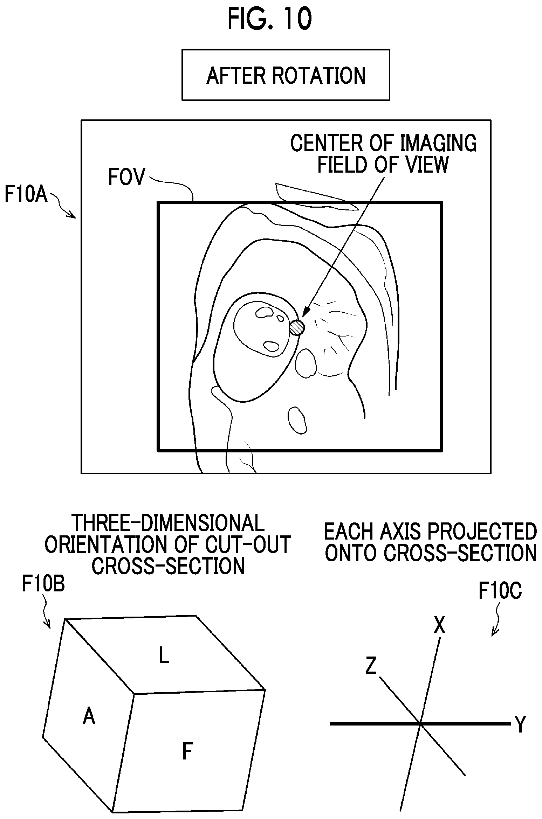

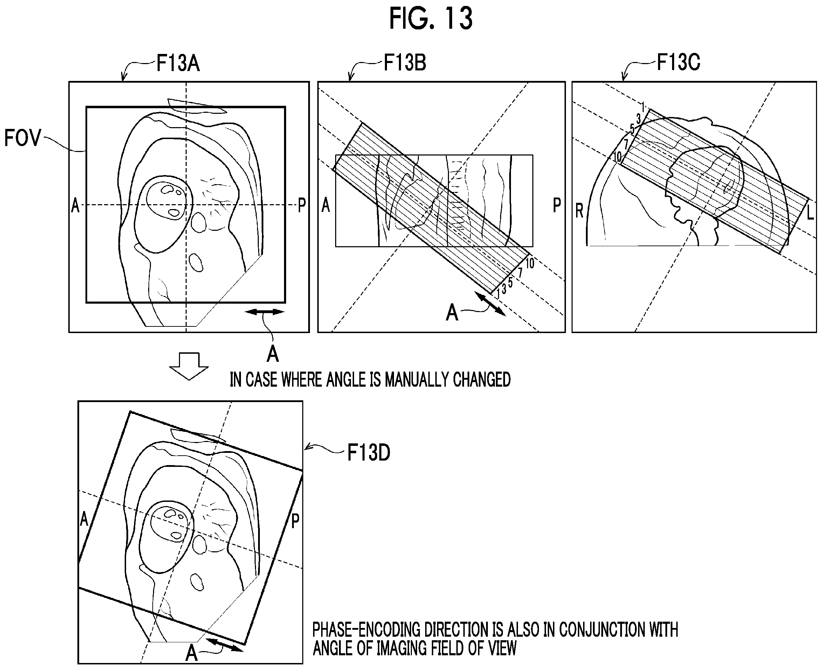

For example, in the case of the left ventricular short-axis image, the cross-sectional image is output at an angle at which the horizontal direction of the cross-sectional image is parallel to a straight line obtained by projecting the AP axis onto the cross-sectional image (see Fig. 10 and an image F13A of Fig. 13). Consequently, the cross-sectional image is displayed with the longitudinal direction of the region of the structures including the heart within the cross-section being substantially parallel to the vertical direction of the cross-sectional image, and the region of the structures falls within the imaging field of view FOV in the horizontal direction, which is the phase-encoding direction. In a frequency-encoding direction perpendicular to the phase-encoding direction, the structures exist outside the imaging field of view FOV, but since artifacts are less likely to occur in the frequency-encoding direction as compared with the phase-encoding direction, the presence of structures outside the imaging field of view FOV in the frequency-encoding direction is allowed.

### Positioning Assistance Method: Specific Example in Case of Setting Imaging Position of Left Ventricular Short-Axis Image

Hereinafter, a more specific example will be described for the case of the left ventricular short-axis image.

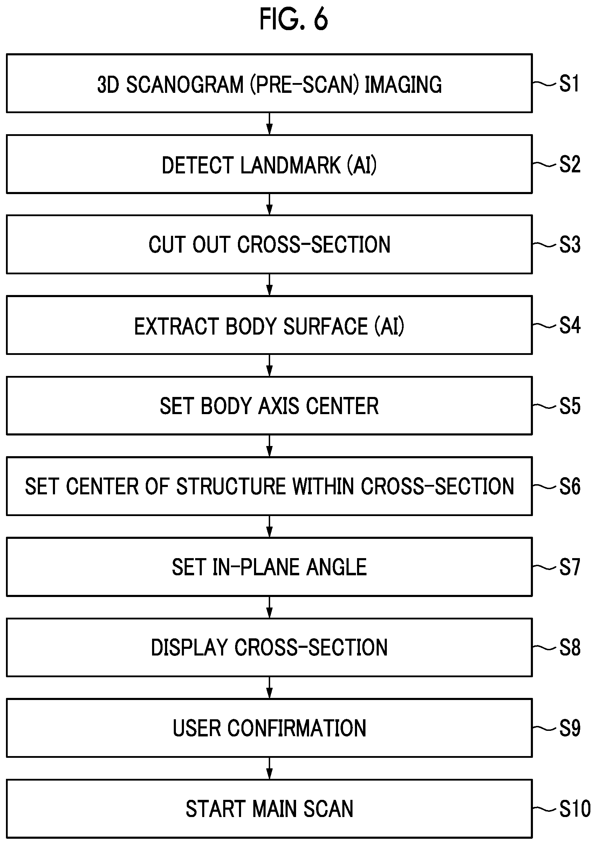

Fig. 6 is a flowchart showing an example of a case where the imaging position of the left ventricular short-axis image is set in the MRI apparatus 10. The method shown in the flowchart of Fig. 6 is an example of the positioning assistance method according to the present disclosure.

In step S1, the processor 202 executes 3D scanogram imaging prior to the main imaging to acquire the 3D scanogram image including the heart of the subject 130. The 3D scanogram image may be, for example, a multi-slice image of the axial cross-section including a three-dimensional region covering the entire heart of the subject 130 or may be an isotropically processed three-dimensional image generated by performing interpolation processing based on the multi-slice image such that data in each axis direction of the three axes of the apparatus is evenly spaced at equal intervals. The 3D scanogram image is an example of a three-dimensional image in the present disclosure.

In step S2, the processor 202 detects the landmark of the heart from the acquired 3D scanogram image. The landmark is a site that serves as a reference point of an anatomical form, and includes, for example, positions such as the apex of the heart, the mitral valve, the long axis, the left atrium, and the tricuspid valve. The long axis of the heart may be obtained as a straight line connecting the apex end of the mitral valve and the apex of the heart.

For example, artificial intelligence (AI) is used for the detection processing of the landmark. That is, the processor 202 may execute processing of detecting the landmark of the heart from the 3D scanogram image using an Al model that has been trained through machine learning. Note that a configuration may be employed in which a plurality of landmarks are identified and detected by one AI model, or a configuration may be employed in which a plurality of Al models are used, with each Al model detecting a different landmark.

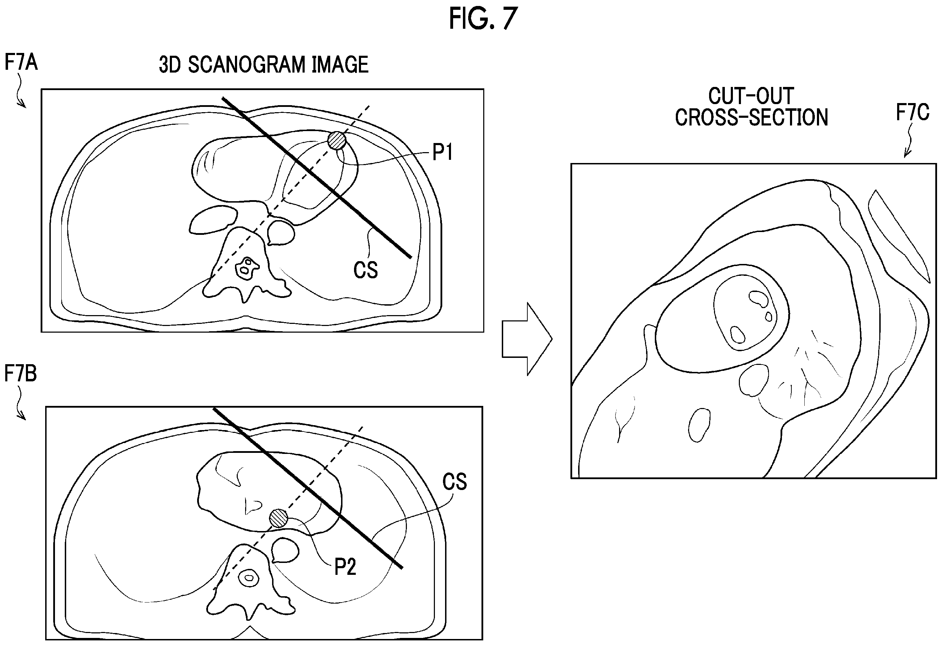

In step S3, the processor 202 determines a cutout position (cross-section position) of the cross-section from the 3D scanogram image using the detected landmark. By determining the cross-section, a normal line perpendicular to the cross-section is determined. Then, the processor 202 cuts out the cross-section from the 3D scanogram image at the determined cross-section position to generate the cross-sectional image (see Fig. 7).

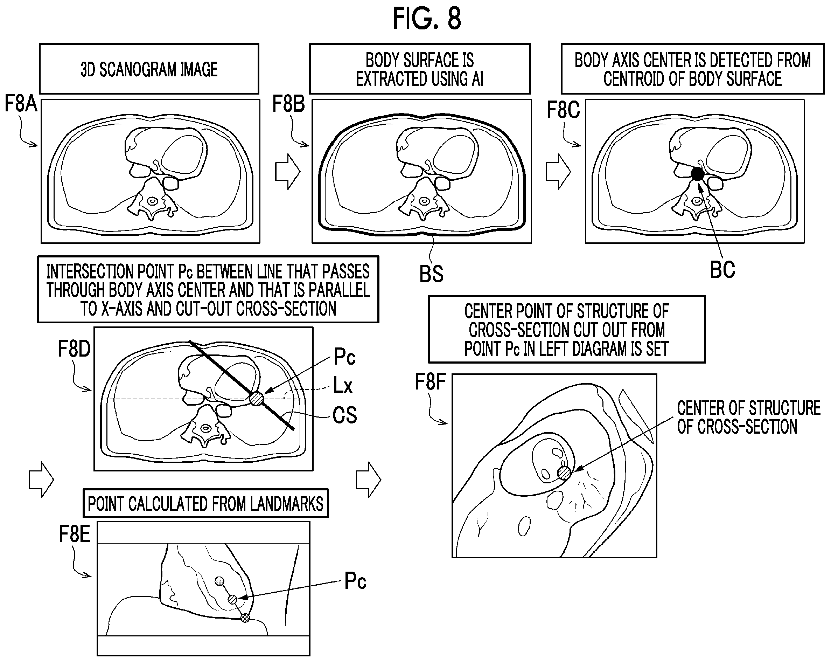

In step S4, the processor 202 extracts a body surface of the subject 130 from the 3D scanogram image (see an image F8A and an image F8B in Fig. 8). The processor 202 may execute processing of extracting the body surface using an Al model that has been trained through machine learning.

In step S5, the processor 202 calculates the centroid of the body surface based on the extracted body surface and sets a body axis center from the centroid of the body surface (see an image F8C in Fig. 8).

In step S6, the processor 202 calculates a center position of the structure within the cross-section based on the body axis center and sets a positional relationship between the imaging field of view and the cross-section such that the center of the imaging field of view matches the center of the structure within the cross-section (see an image F8F in Fig. 8).

In step S7, the processor 202 sets the in-plane angle for determining the horizontal (lateral) direction and the vertical (longitudinal) direction of the cross-sectional image cut out in step S3. In a case of determining the horizontal direction and the vertical direction of the cross-sectional image, the processor 202 sets a direction of one side of any of the horizontal direction or the vertical direction of the cross-sectional image such that any one of the horizontal direction or the vertical direction of the cross-sectional image is parallel to a straight line obtained by projecting any one of the three axes of the apparatus (three orthogonal axes in a case of capturing the 3D scanogram image) onto the cross-sectional image. The direction of the one side is referred to as a "direction of a first side". Then, a direction of the other one side in the cross-sectional image (referred to as a "direction of a second side") is set as a direction perpendicular to the direction of the first side on the plane of the cross-section.

By setting the direction of the first side, the direction of the second side perpendicular to the direction of the first side is inevitably determined. By setting the direction of the first side, the in-plane angle for determining the horizontal (lateral) direction and the vertical (longitudinal) direction of the cross-sectional image cut out in step S3 is specified. Setting the direction of the first side and setting the in-plane angle of the cross-sectional image are substantially equivalent.

The three axes of the apparatus are examples of three axes that form the three-dimensional orthogonal coordinate system of the real space including the imaging space 18. The three axes of the apparatus during 3D scanogram image capturing are associated with the HF axis, the AP axis, and the RL axis of the subject 130 on the patient table 14. For example, in a case where the posture of the subject 130 is a supine posture with head-first orientation, the HF axis matches the Z direction, the AP axis matches the Y direction, and the RL axis matches the X direction.

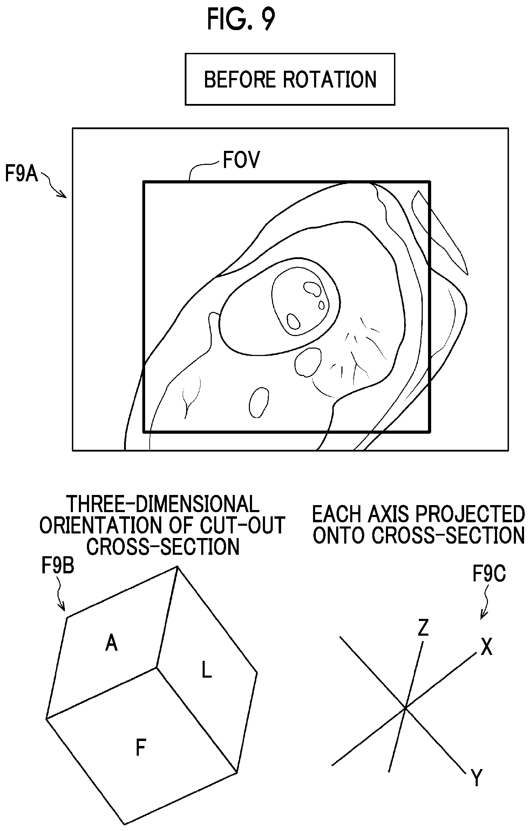

In the case of the left ventricular short-axis image, the processor 202 sets the in-plane angle such that the horizontal direction of the cross-sectional image is parallel to the straight line obtained by projecting the AP axis onto the cross-sectional image, and rotates the cross-sectional image in accordance with the set in-plane angle (see Figs. 9 to 12).

In step S8, the processor 202 displays the cross-sectional image rotated in accordance with the in-plane angle set in step S7 on the display device 214. The processor 202 automatically sets the imaging field of view FOV for the cross-sectional image and displays a frame (hereinafter, referred to as an imaging field of view frame) indicating the region of the imaging field of view FOV in a superimposed manner on the cross-sectional image. The "automatic setting" herein may be the presentation of recommended settings proposed by the automatic calculation of the automatic positioning function implemented in the MRI apparatus 10, and the final determination (finalization) of the imaging field of view FOV may be left to the user.

The processor 202 may automatically generate a display image with the imaging field of view frame based on the automatic setting superimposed on the cross-sectional image and display the display image on the display device 214. In addition, the processor 202 may accept an instruction from the user regarding whether or not to display the imaging field of view frame via the input device 212 and may switch between display and non-display of the imaging field of view frame according to the accepted instruction.

For example, a configuration may be employed in which, in the default setting, automation is set up to the point where the imaging field of view frame based on the automatic setting is displayed in a superimposed manner on the cross-sectional image, and the setting can be changed to the non-display of the imaging field of view frame based on the user's selection.

On the screen of the display device 214, the imaging field of view frame is displayed together with the cross-sectional image, thereby prompting the user for confirmation.

In step S9, the user confirms the cross-sectional image and the imaging field of view FOV and inputs an instruction to execute the setting of the imaging position or to change the setting. The processor 202 accepts the instruction from the user and provides the UI in a case where the setting is manually changed (see Fig. 13). The processor 202 accepts an instruction such as fine adjustment of the imaging position via the UI.

After the user confirms the setting in step S9, the processor 202 transitions to step S10.

In step S10, the processor 202 executes the main imaging under the set imaging conditions.

### Detailed Description of Step S3: Specific Example of Cross-Section Cutout Processing

Fig. 7 is an explanatory diagram showing an example of cross-section cutout processing in step S3 of Fig. 6. An image F7A and an image F7B shown on the left side of Fig. 7 are image examples of a representative cross-section of the 3D scanogram image. A point P1 in the image F7A is a point of the apex of the heart detected by the landmark detection processing (step S2). A point P2 in the image F7B is a point of the left atrium detected by the landmark detection (step S2).

The processor 202 sets a cross-section CS of the left ventricular short-axis image based on a plurality of points P1 and P2 detected by the landmark detection processing.

An image F7C shown on the right side of Fig. 7 is an example of a cross-sectional image of the cross-section CS cut out from the 3D scanogram image.

Detailed Description of Steps S4 to S6: Method of Setting Center of Imaging Field of View of Left Ventricular Short-Axis Image

Fig. 8 is an explanatory diagram showing an example of a method of setting the center of an imaging field of view of the left ventricular short-axis image. Fig. 8 shows a flow of processing of step S4 to step S6 of Fig. 6 together with image examples. The image F8A shown in the upper left of Fig. 8 is an example of the 3D scanogram image acquired in step S1. The image F8B shows an example in which a body surface BS is extracted from the 3D scanogram image by the processing of step S4. The processor 202 extracts the body surface BS from the 3D scanogram image using the Al (see the image F8B).

The image F8C shows an example in which a body axis center BC is detected by the processing of step S5. The processor 202 detects the body axis center BC from the centroid of the body surface BS.

Further, the processor 202 obtains an intersection point Pc between a line Lx, which passes through the body axis center BC and is parallel to the X-axis, and the cross-section CS cut out in step S3, as shown in an image F8D. The processor 202 can calculate the intersection point Pc from a plurality of landmarks detected in step S2 as shown in an image F8E.

Then, the processor 202 sets the center point of the structure in the cross-section CS from the points Pc shown in the images F8D and F8E (see the image F8F).

By matching the center point of the structure obtained in this manner with the center of the imaging field of view, the structure outside the imaging field of view, which is a factor of artifacts, can be minimized.

### Description of Processing Content of Step S7: In-Plane Angle Setting Method

Figs. 9 and 10 show examples of setting the in-plane angle of the left ventricular short-axis image. Fig. 9 shows a state of the cross-sectional image before rotation, which has been cut out from the 3D scanogram image, and Fig. 10 shows a state of the cross-sectional image after rotation, which has been rotated at the appropriately set in-plane angle.

A cross-sectional image F9A shown in Fig. 9 is an image example of the cross-section cut out from the 3D scanogram image by the processing of step S3. A cube F9B shown in the lower left of Fig. 9 represents a three-dimensional orientation of the cross-section obtained by cutting out the cross-sectional image F9A. That is, assuming that the cross-section obtained by cutting out the cross-sectional image F9A (the cross-section on which the cross-sectional image F9A appears) is a plane parallel to the paper plane of Fig. 9, the cube F9B represents the orientation of volume data of the 3D scanogram image from which this cross-section is obtained. The symbols "A", "F", and "L" attached to each surface of the cube F9B indicate the first letters of "anterior", "foot", and "left", respectively. Although not shown in Fig. 9, a surface of the cube F9B facing the "L" surface is "R", a surface facing the "A" surface is "P", and a surface facing the "F" surface is "H".

The cube F9B shows how the two-dimensional cross-section on which the cross-sectional image F9A appears corresponds to the rotation of the 3D volume data from the original 3D scanogram image.

An intersection diagram F9C in which three straight lines shown in the lower right of Fig. 9 intersect each other represents a straight line obtained by projecting each axis (the X-axis, the Y-axis, and the Z-axis) of the three axes of the apparatus onto the cross-section obtained by cutting out the cross-sectional image F9A. The three axes of the apparatus correspond to three axes of the body axis of the subject 130, and for example, in a case where the posture of the subject 130 is a supine posture with head-first orientation, the X-axis corresponds to the RL axis, the Y-axis corresponds to the AP axis, and the Z-axis corresponds to the HF axis.

The straight line of each axis projected onto the cross-section cut out from the 3D scanogram image may be understood as a straight line obtained by projecting each axis of the RL axis, the AP axis, and the HF axis of the cube F9B onto the cross-sectional image.

As shown in the intersection diagram F9C, the straight line of each axis projected onto the cross-section is not parallel to the horizontal direction or the vertical direction of the cross-sectional image F9A. That is, in a state before the image is rotated, none of the RL direction, the AP direction, and the HF direction projected onto the cross-sectional image F9A are aligned with the longitudinal direction or the lateral direction of the cross-sectional image F9A.

As described in Fig. 5, in a case where the cross-sectional image F9A is output at the angle shown in Fig. 9, structures exist outside the imaging field of view FOV in the phase-encoding direction (here, the horizontal direction of the cross-sectional image F9A), causing the occurrence of aliasing artifacts.

Therefore, in the MRI apparatus 10 according to the present embodiment, instead of outputting the cross-sectional image F9A as shown in Fig. 9, the in-plane angle is set such that the right-left direction (horizontal direction) on the cross-sectional image is parallel to the straight line obtained by projecting the AP axis onto the cross-sectional image, and a cross-sectional image F10A is output, as shown in Fig. 10.

The cross-sectional image F10A shown in Fig. 10 is an example of the cross-sectional image after rotation, which is generated by the processing of step S7, and is an example of the image displayed on the display device 214 by the processing of the cross-section display in step S8.

A cube F10B shown in the lower left of Fig. 10 represents a three-dimensional orientation of the cross-section obtained by cutting out the cross-sectional image F10A and shows how the two-dimensional cross-section on which the cross-sectional image F10A appears corresponds to the rotation of the 3D volume data from the original 3D scanogram image. An intersection diagram F10C in which three straight lines shown in the lower right of Fig. 10 intersect each other represents a straight line obtained by projecting each axis of the three axes of the apparatus onto the cross-sectional image obtained by cutting out the cross-sectional image F10A.

As shown in Fig. 10, the cross-sectional image F10A is obtained by in-plane rotation of the cross-sectional image F9A of Fig. 9 such that the straight line obtained by projecting the Y-axis (AP axis) onto the cross-sectional image and one side of the cross-section in the right-left direction (a side of the cross-sectional image F10A in the horizontal direction) are parallel to each other. In the example of Fig. 10, the Y-axis (AP axis) is an example of "one axis selected from among three axes" in the present disclosure.

As is clear from the comparison between Figs. 9 and 10, by rotating the cross-sectional image F9A before rotation in a counterclockwise direction with the normal line of the cross-section as the rotation axis, the cross-sectional image F10A as shown in Fig. 10 can be obtained.

That is, as in Fig. 9, in a case where each axis of the three axes of the apparatus is projected on the cross-sectional image and the cross-sectional image F9A is rotated, each projected straight line of the three axes is also rotated together with the cross-sectional image F9A. By rotating the image until a specific one axis among the rotated straight lines of the three axes, the straight line of the Y-axis, becomes parallel to the horizontal direction of the cross-sectional image, the cross-sectional image F10A as shown in Fig. 10 can be obtained.

Additionally, the processor 202 displays the imaging field of view frame indicating the region of the automatically set imaging field of view FOV as an overlay on the cross-sectional image F10A. The imaging field of view frame based on the automatic setting is displayed on the screen of the display device 214 as an upright quadrangular frame line, similar to that shown in Fig. 5. That is, the imaging field of view frame presented on the cross-sectional image F10A by the automatic positioning function according to the present embodiment is a quadrangle surrounded by sides along the longitudinal direction and the lateral direction that match the longitudinal direction and the lateral direction of the screen of the display device 214. The horizontal direction of the cross-sectional image F10A shown in Fig. 10 is the phase-encoding direction.

By displaying the cross-sectional image F10A on which the imaging field of view frame is superimposed, the user can confirm the setting of the imaging position on the screen of the display device 214.

In Figs. 9 and 10, an example is shown in which the cut-out cross-section includes a component in the AP direction, but in the case of the left ventricular short-axis image, the cut-out cross-section may not include the component in the AP direction depending on the position of the cut-out cross-section.

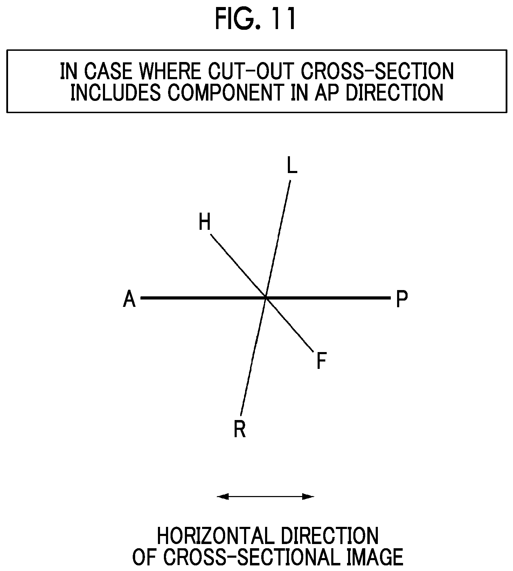

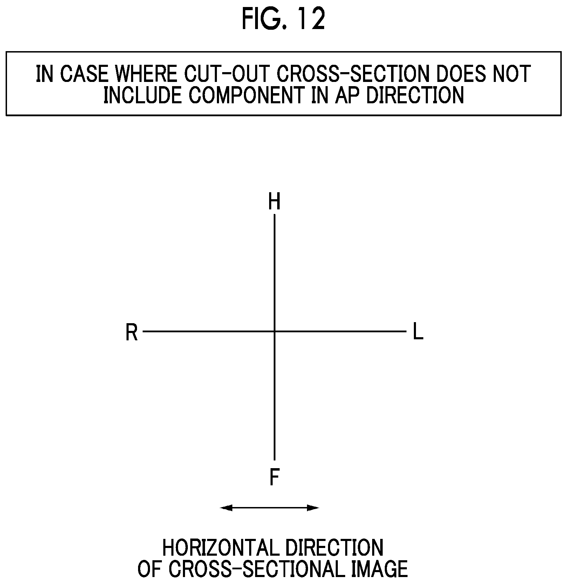

In a case where the cut-out cross-section includes the component in the AP direction, as shown in Fig. 11, the processor 202 outputs the cross-sectional image at an angle at which the horizontal direction of the cross-sectional image is parallel to the straight line obtained by projecting the AP axis onto the cross-sectional image. On the other hand, in a case where the cut-out cross-section does not include the AP component, as shown in Fig. 12, the processor 202 outputs the cross-sectional image at an angle at which the horizontal direction and the vertical direction of the cross-sectional image are set to the same horizontal direction and the same vertical direction as those of the coronal cross-section. That is, in a case where the cut-out cross-section does not include the AP component, the cross-sectional image is output at an angle at which the horizontal direction of the cross-sectional image is parallel to the straight line obtained by projecting the RL axis onto the cross-sectional image and the vertical direction of the cross-sectional image is parallel to the straight line obtained by projecting the HF axis onto the cross-sectional image.

### Specific Example of Steps S8 and S9: Display Example of Cross-Sectional Image and Method of Manually Changing Imaging Field of View

Fig. 13 shows an example of the UI in a case of performing the cross-section display of the left ventricular short-axis image. Images F13A, F13B, and F13C shown in the upper part of Fig. 13 are setting examples of recommended imaging positions automatically calculated based on the 3D scanogram image. A bidirectional arrow A in the drawing indicates the phase-encoding direction.

The image F13A is the same as the cross-sectional image F10A shown in Fig. 10. The image F13B is an example of an image in which the positions of a plurality of imaging cross-sections set in correspondence with the imaging field of view FOV are displayed as an overlay on the vertical long-axis image. The image F13C is an example of an image in which the positions of the plurality of imaging cross-sections set in correspondence with the imaging field of view FOV are displayed as an overlay on the horizontal long-axis image.

The plurality of images F13A, F13B, and F13C may be displayed on one screen at the same time on the screen of the display device 214, or each of the images may be individually displayed based on the user's selection. The MRI apparatus 10 accepts an instruction to change the imaging field of view FOV via the input device 212. For example, the user can perform operations such as dragging the frame line of the imaging field of view FOV overlaid and displayed within the image F13A on the screen on which the image F13A is displayed, to rotate, move, or transform the frame line. In addition, the user can finely adjust the imaging field of view FOV by designating the rotation angle, the movement amount, or the transformation parameter through numerical value input via the input device 212.

An image F13D is an example of a case where the user manually changes the angle of the imaging field of view FOV. As in the image F13D, in a case where the user manually changes the angle of the imaging field of view FOV, the phase-encoding direction is also changed in conjunction with the angle of the imaging field of view FOV.

The user confirms the setting of the imaging position on the screen, confirms that the setting is appropriate, finalizes the setting content, and then transitions to the main imaging. The cross-sectional image of the reference cross-section and the information on the imaging field of view FOV, with the imaging position setting finalized, are stored in the storage 206 in association with an examination image for diagnosis captured by the main imaging. The storage 206 may be a storage device of an image management system such as a picture archiving and communication system (PACS).

### Setting Example of Horizontal Direction or Vertical Direction of Cross-Sectional Image Corresponding to Type of Reference Cross-Section

In the cardiac MRI examination, imaging of a plurality of reference cross-sections is usually performed in a single examination. It is preferable that the processor 202 sets, for at least one of the plurality of reference cross-sections, and preferably for all of the cross-sectional images of the plurality of reference cross-sections, the horizontal direction or the vertical direction of the cross-sectional image with reference to the axes of the apparatus using the same method as the example of the left ventricular short-axis image mentioned above, and generates a display image.

Preferred setting examples of the horizontal direction or the vertical direction for the reference cross-sectional images other than the left ventricular short-axis image are as follows.

### In Case of Horizontal Long-Axis Image

In a case where the cross-section cut out from the 3D scanogram image includes a component in the RL direction when the horizontal long-axis image is generated, the processor 202 outputs the cross-sectional image at an angle at which the horizontal direction of the cross-sectional image is parallel to the straight line obtained by projecting the RL axis onto the cross-sectional image.

On the other hand, in a case where the cut-out cross-section does not include an RL component, the processor 202 outputs the cross-sectional image at an angle at which the horizontal direction and the vertical direction of the cross-sectional image are set to the same horizontal direction and the same vertical direction as those of the sagittal cross-sectional image. That is, in a case where the cut-out cross-section does not include an RL component, the cross-sectional image is output at an angle at which the horizontal direction of the cross-sectional image is parallel to the straight line obtained by projecting the AP axis onto the cross-sectional image and the vertical direction of the cross-sectional image is parallel to the straight line obtained by projecting the HF axis onto the cross-sectional image.

### In Case of Four-Chamber Long-Axis Image

In a case where the cross-section cut out from the 3D scanogram image includes a component in the RL direction when the four-chamber long-axis image is generated, the processor 202 outputs the cross-sectional image at an angle at which the horizontal direction of the cross-sectional image is parallel to the straight line obtained by projecting the RL axis onto the cross-sectional image.

On the other hand, in a case where the cut-out cross-section does not include an RL component, the processor 202 outputs the cross-sectional image at an angle at which the horizontal direction and the vertical direction of the cross-sectional image are set to the same horizontal direction and the same vertical direction as those of the sagittal cross-sectional image.

### In Case of Two-Chamber Long-Axis Image

In a case where the cross-section cut out from the 3D scanogram image includes a component in the HF direction (head-foot direction) when the two-chamber long-axis image is generated, the processor 202 outputs the cross-sectional image at an angle at which the vertical direction of the cross-sectional image is parallel to the straight line obtained by projecting the HF axis onto the cross-sectional image.

On the other hand, in a case where the cut-out cross-section does not include an HF component, the processor 202 outputs the cross-sectional image at an angle at which the horizontal direction and the vertical direction of the cross-sectional image are set to the same horizontal direction and the same vertical direction as those of the axial cross-sectional image. That is, in a case where the cut-out cross-section does not include an HF component, the cross-sectional image is output at an angle at which the horizontal direction of the cross-sectional image is parallel to the straight line obtained by projecting the RL axis onto the cross-sectional image and the vertical direction of the cross-sectional image is parallel to the straight line obtained by projecting the AP axis onto the cross-sectional image.

### In Case of Three-Chamber Long-Axis Image

In a case where the cross-section cut out from the 3D scanogram image includes a component in the AP direction when the three-chamber long-axis image is generated, the processor 202 outputs the cross-sectional image at an angle at which the horizontal direction of the cross-sectional image is parallel to the straight line obtained by projecting the AP axis onto the cross-sectional image.

On the other hand, in a case where the cut-out cross-section does not include an AP component, the processor 202 outputs the cross-sectional image at an angle at which the horizontal direction and the vertical direction of the cross-sectional image are set to the same horizontal direction and the same vertical direction as those of the coronal cross-sectional image.

### In Case of Vertical Long-Axis Image

Since the cross-section of the vertical long-axis image is defined as a cross-section parallel to the Z direction (HF direction) of the axes of the apparatus, the Z direction need only be set as the vertical (longitudinal) direction of the cross-sectional image, and rotation is not required.

### Functional Configuration of Information Processing Apparatus 200

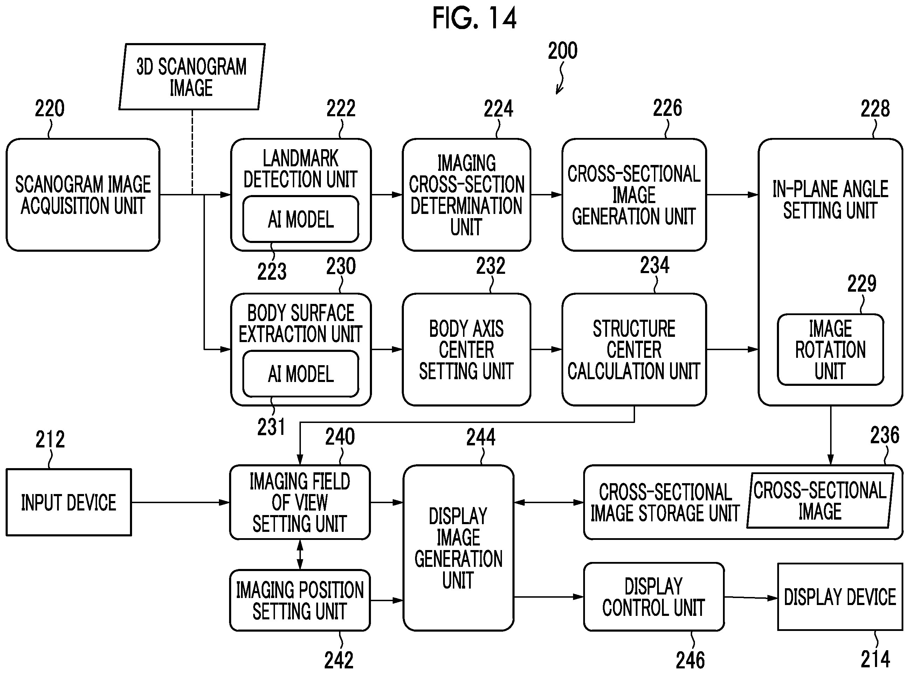

Fig. 14 is a block diagram showing a functional configuration of the information processing apparatus 200. The information processing apparatus 200 includes a scanogram image acquisition unit 220, a landmark detection unit 222, an imaging cross-section determination unit 224, a cross-sectional image generation unit 226, an in-plane angle setting unit 228, a body surface extraction unit 230, a body axis center setting unit 232, a structure center calculation unit 234, a cross-sectional image storage unit 236, an imaging field of view setting unit 240, an imaging position setting unit 242, a display image generation unit 244, and a display control unit 246.

The scanogram image acquisition unit 220 acquires the 3D scanogram image captured by executing the scanogram imaging. The landmark detection unit 222 includes a trained Al model 223 and detects the landmark of the heart from the 3D scanogram image using the Al model 223. The Al model 223 may be, for example, an image recognition model that is configured using a convolutional neural network and that is trained to perform an object detection task. The Al model 223 is an example of a "first artificial intelligence model" in the present disclosure.

The imaging cross-section determination unit 224 determines the position of the reference cross-section as the imaging cross-section based on the landmark detected by the landmark detection unit 222. The imaging cross-section determination unit 224 determines the cross-section including a plurality of landmarks. The imaging cross-section determination unit 224 determines, for example, the positions of the six types of reference cross-sections mentioned above.

The cross-sectional image generation unit 226 generates the cross-sectional image of the reference cross-section from the 3D scanogram image. That is, the cross-sectional image generation unit 226 generates the cross-sectional image appearing on the cross-section in a case where the 3D scanogram image is cut at the position of the reference cross-section determined by the imaging cross-section determination unit 224.

The in-plane angle setting unit 228 sets an image rotation angle for determining the horizontal direction or the vertical direction of the cross-sectional image cut out from the 3D scanogram image. The in-plane angle setting unit 228 sets an angle of rotation of the cross-sectional image according to the type of the reference cross-section such that the horizontal direction or the vertical direction of the cross-sectional image is parallel to the straight line obtained by projecting one axis selected from the three axes of the apparatus onto the cross-sectional image. The in-plane angle setting unit 228 includes an image rotation unit 229 that performs image rotation processing. The image rotation unit 229 rotates the cross-sectional image at the set angle and generates the cross-sectional image in which the horizontal direction or the vertical direction of the cross-sectional image is parallel to the straight line obtained by projecting the one axis selected from the three axes of the apparatus onto the cross-sectional image. In this manner, the cross-sectional image that has been adjusted in a correct orientation is stored in the cross-sectional image storage unit 236. The cross-sectional image storage unit 236 is a storage area of the memory 204 and/or the storage 206.

The body surface extraction unit 230 includes a trained Al model 231 and extracts the body surface BS of the subject 130 from the 3D scanogram image using the Al model 231. The Al model 231 may be, for example, an image recognition model that is configured using a convolutional neural network and that is trained to perform a region classification (semantic segmentation) task. The Al model 231 is an example of a "second artificial intelligence model" in the present disclosure.

The body axis center setting unit 232 sets the body axis center based on an extraction result of the body surface by the body surface extraction unit 230. The structure center calculation unit 234 calculates the center point of the structure within the cross-section based on the body axis center and the cut-out cross-section.

The information on the center point of the structure is transmitted to the imaging field of view setting unit 240. The imaging field of view setting unit 240 provides the setting of the recommended imaging field of view FOV according to the type of the reference cross-section. The imaging field of view setting unit 240 sets the positional relationship between the cross-sectional image and the imaging field of view such that the center of the structure within the cross-section and the center of the imaging field of view match each other. Additionally, the imaging field of view setting unit 240 can accept a change of the setting of the imaging field of view via the input device 212 and change the setting of the imaging field of view in accordance with the accepted change instruction.

The imaging position setting unit 242 sets the imaging position of each slice in the main imaging based on the setting of the imaging field of view setting unit 240. The display image generation unit 244 performs processing of generating various display images to be displayed on the display device 214. The display control unit 246 generates a display signal required for display output to the display device 214 and performs the display control of the display device 214. The image generated by the display image generation unit 244 is displayed on the display device 214 via the display control unit 246.

### Regarding Hardware Configuration of Each Processing Unit

The hardware structure of the processing unit of the information processing apparatus 200 that executes various types of processing, such as the scanogram image acquisition unit 220, the landmark detection unit 222, the imaging cross-section determination unit 224, the cross-sectional image generation unit 226, the in-plane angle setting unit 228, the image rotation unit 229, the body surface extraction unit 230, the body axis center setting unit 232, the structure center calculation unit 234, the imaging field of view setting unit 240, the imaging position setting unit 242, the display image generation unit 244, and the display control unit 246, is, for example, various processors as described below.

The various processors include a CPU that is a general-purpose processor which functions as various processing units by executing programs, a GPU that is a processor specialized for image processing, a programmable logic device (PLD) that is a processor whose circuit configuration can be changed after manufacturing, such as a field programmable gate array (FPGA), a dedicated electrical circuit that is a processor having a dedicated circuit configuration designed to execute specific processing, such as an application specific integrated circuit (ASIC), and the like.

One processing unit may be configured using one of these various processors or may be configured using two or more processors of the same type or different types. For example, one processing unit may be configured using a plurality of FPGAs, or a combination of a CPU and an FPGA or a combination of a CPU and a GPU. Alternatively, a plurality of processing units may be configured using one processor. As an example of configuring a plurality of processing units using one processor, first, there is an aspect in which one processor is configured using a combination of one or more CPUs and software and this processor functions as a plurality of processing units, as represented by computers such as clients or servers. Second, there is an aspect in which a processor that implements the functions of an entire system, which includes a plurality of processing units, with a single integrated circuit (IC) chip is used, as represented by systems on chip (SoC) and the like. In this way, the various processing units are configured using one or more of the above-described various processors as the hardware structure.

Further, the hardware structure of these various processors is, more specifically, an electrical circuit (circuitry) combined with circuit elements such as semiconductor elements.

### Regarding Program for Operating Computer

A program for causing a computer to implement a part or all of the processing functions of the information processing apparatus 200 can be recorded on a computer-readable medium, which is a non-transitory information storage medium such as an optical disk, a magnetic disk, a semiconductor memory, or other tangible media, and the program can be provided through this information storage medium.

In addition, instead of the aspect in which the program is stored in such a tangible and non-transitory computer-readable medium and then provided, program signals can also be provided as a download service using telecommunication lines such as the Internet.

Further, a part or all of the processing functions of the information processing apparatus 200 may be implemented using cloud computing or may be provided as software as a service (SaaS).

### Advantages of the Present Embodiment

With the MRI apparatus 10 according to the present embodiment, the following advantages are provided.
[1] In a case of generating the cross-sectional image of the reference cross-section from the 3D scanogram image, the processor 202 sets the horizontal direction or the vertical direction of the cross-sectional image with reference to the straight line obtained by projecting the axis of the apparatus, which defines the three-dimensional orthogonal coordinate system of the real space including the imaging space 18, onto the cross-sectional image. Therefore, regardless of the subject, the structures within the cross-section are displayed on the screen of the display device 214 at the same angle.
[2] It is possible to output the cross-sectional image at an angle at which the structure outside the imaging field of view in the phase-encoding direction, which is a factor of artifacts, is reduced. As a result, aliasing artifacts can be suppressed.
[3] In particular, by matching the center of the structure within the cross-section with the center of the imaging field of view, the structure outside the imaging field of view, which is a factor of artifacts, can be minimized.
[4] According to the present embodiment, the position of the cross-section and the imaging range recommended as the imaging position of the main imaging are automatically calculated from the 3D scanogram image, and the information on the imaging field of view FOV based on the automatic setting is provided together with the cross-sectional image. This enables the efficient positioning of the imaging position for the cardiac MRI examination.
[5] On the screen of the display device 214 that displays the cross-sectional image generated from the 3D scanogram image, the frame (imaging field of view frame) indicating the region of the imaging field of view FOV by the automatic positioning is presented as an upright quadrangular frame that matches the longitudinal and lateral orientations of the screen. As a result, the user can easily understand the positional relationship between the imaging field of view FOV and the structure within the cross-section.

### Modification Example 1

A configuration is also possible in which steps S4 to S6 in Fig. 6 are omitted.

### Modification Example 2

The landmark detection processing of step S2 is not limited to the configuration in which Al is used, and an image processing method such as pattern recognition or feature point extraction may be applied instead of or in combination with Al. Similarly, the body surface extraction processing of step S4 is not limited to the configuration in which Al is used, and for example, a known method of extracting a region with an image density equal to or higher than a reference density as a subject region and extracting a boundary thereof as the body surface may be applied, instead of or in combination with Al.

### Modification Example 3

In the above-mentioned embodiment, an example has been described in which the display of the imaging field of view frame is automatically performed at the same time as the cross-sectional image in the cross-section display processing of step S8. However, the present disclosure is not limited to this example. For example, automation may be set up to the point where the cross-sectional image is displayed without the imaging field of view frame, and after the cross-sectional image display, the imaging field of view frame may be displayed on the cross-sectional image by receiving an input of an instruction to set the imaging field of view FOV through the UI.

### Modification Example 4

In the above-mentioned embodiment, as in Fig. 10, the horizontal direction (lateral direction) on the image in a case where the imaging field of view FOV is set is set as the phase-encoding direction. However, the vertical direction (longitudinal direction) on the image can be set as the phase-encoding direction. In this case, the processor 202 sets the angle of rotation of the cross-sectional image such that the structure outside the imaging field of view FOV in the longitudinal direction, which is the phase-encoding direction, is minimized, and preferably such that no structure exists outside the imaging field of view FOV in the phase-encoding direction, and adjusts the orientation of the cross-sectional image through the rotation processing.

### Others

The present disclosure is not limited to the above-mentioned embodiment, and various modifications are possible within the scope of the technical concept of the present disclosure without departing from its gist.

### Explanation of References

10: MRI apparatus
12: gantry
14: patient table
15: top plate
16: leg portion
18: imaging space
102: static magnetic field generating magnet
104: gradient magnetic field coil
106: RF transmit coil
110: receive coil
112: receiver
114: gradient magnetic field power supply
116: high-frequency magnetic field generator
118: sequencer
120: control unit
122: operation unit
130: subject
200: information processing apparatus
202: processor
204: memory
206: storage
208: input/output interface
210: bus
212: input device
214: display device
220: scanogram image acquisition unit
222: landmark detection unit
223: Al model
224: imaging cross-section determination unit
226: cross-sectional image generation unit
228: in-plane angle setting unit
229: image rotation unit
230: body surface extraction unit
231: Al model
232: body axis center setting unit
234: structure center calculation unit
236: cross-sectional image storage unit
240: imaging field of view setting unit
242: imaging position setting unit
244: display image generation unit
246: display control unit
A: bidirectional arrow
BC: body axis center
BS: body surface
CS: cross-section
F4A: left diagram
F4B: right diagram
F7A, F7B, F7C: image
F8A, F8B, F8C, F8D, F8E, F8F: image
F9A: cross-sectional image
F9B: cube
F9C: intersection diagram
F10A: cross-sectional image
F10B: cube
F10C: intersection diagram
F13A, F13B, F13C, F13D: image
FOV: imaging field of view
Lx: line
P1: point
P2: point
Pc: intersection point
S1 to S10: steps of positioning assistance method

## Claims

1. A magnetic resonance imaging apparatus (10) that generates an image of a subject (130) based on nuclear magnetic resonance, the apparatus comprising:
a processor (202); and
a memory (204),
wherein the processor (202) is configured to:
acquire a three-dimensional image including a heart of the subject (130) through scanogram imaging executed prior to main imaging;
detect a landmark of the heart from the three-dimensional image;
set a position of a cross-section (CS) based on the landmark;
generate a cross-sectional image (F9A, F10A) appearing on the cross-section (CS) from the three-dimensional image;
set a horizontal direction or a vertical direction of the cross-sectional image (F9A, F10A) such that the horizontal direction or the vertical direction of the cross-sectional image (F9A, F10A) is parallel to a straight line obtained by projecting one axis selected from among three axes that form a three-dimensional orthogonal coordinate system of a real space onto the cross-sectional image (F9A, F10A); and
store the cross-sectional image (F9A, F10A) in the memory (204).

2. The magnetic resonance imaging apparatus (10) according to claim 1,
wherein the processor (202) is configured to:
rotate the cross-sectional image (F9A, F10A) at an angle at which the horizontal direction or the vertical direction of the cross-sectional image (F9A, F10A) is parallel to the straight line projected onto the cross-sectional image (F9A, F10A); and
store the rotated cross-sectional image (F9A, F10A) in the memory (204).

3. The magnetic resonance imaging apparatus (10) according to claim 1 or 2,
wherein the processor (202) is configured to:
generate a display image in which a frame indicating a region of an imaging field of view (FOV) is superimposed on the cross-sectional image (F9A, F10A), and
preferably, the frame indicating the region of the imaging field of view (FOV) displayed on the cross-sectional image (F9A, F10A) is a quadrangular frame surrounded by one set of opposing sides parallel to the horizontal direction of the cross-sectional image (F9A, F10A) and one set of opposing sides parallel to the vertical direction of the cross-sectional image (F9A, F10A).

4. The magnetic resonance imaging apparatus (10) according to any one of claims 1 to 3,
wherein the processor (202) is configured to:
detect the landmark using a first artificial intelligence model that has been trained through machine learning.

5. The magnetic resonance imaging apparatus (10) according to any one of claims 1 to 4, wherein the processor (202) is configured to:
extract a body surface from the three-dimensional image;
set a body axis center based on the extracted body surface;
set a center of a structure within the cross-section (CS) based on the body axis center; and
set the imaging field of view (FOV) for the cross-sectional image (F9A, F10A) such that a center of the imaging field of view (FOV) matches the center of the structure within the cross-section (CS).

6. The magnetic resonance imaging apparatus (10) according to claim 5,
wherein the processor (202) is configured to:
extract the body surface using a second artificial intelligence model that has been trained through machine learning.

7. The magnetic resonance imaging apparatus (10) according to any one of claims 1 to 6,
wherein the horizontal direction or the vertical direction of the cross-sectional image (F9A, F10A) is a phase-encoding direction,
and/or
wherein the cross-section (CS) includes a cross-section (CS) from which at least one of a left ventricular short-axis image, a horizontal long-axis image, a four-chamber long-axis image, a two-chamber long-axis image, or a three-chamber long-axis image is obtained.

8. The magnetic resonance imaging apparatus (10) according to any one of claims 1 to 7,
wherein the cross-section (CS) is any one of a reference cross-section (CS) from which a left ventricular short-axis image is obtained and a reference cross-section from which a three-chamber long-axis image is obtained, and
the processor (202) is configured to:
in a case where the cross-section (CS) includes a component in an AP direction which is an anterior-posterior direction of the subject (130), rotate the cross-sectional image at an angle at which the horizontal direction of the cross-sectional image is parallel to a straight line obtained by projecting an AP axis as the one axis onto the cross-sectional image.

9. The magnetic resonance imaging apparatus (10) according to claim 8,
wherein the processor (202) is configured to:
in a case where the cross-section (CS) does not include the component in the AP direction, set the horizontal direction and the vertical direction of the cross-sectional image to be the same as a horizontal direction and a vertical direction of a coronal cross-sectional image.

10. The magnetic resonance imaging apparatus (10) according to any one of claims 1 to 7,
wherein the cross-section (CS) is any one of a reference cross-section from which a horizontal long-axis image is obtained and a reference cross-section from which a four-chamber long-axis image is obtained, and
the processor (202) is configured to:
in a case where the cross-section includes a component in an RL direction which is a right-left direction of the subject (130), rotate the cross-sectional image at an angle at which the horizontal direction of the cross-sectional image is parallel to a straight line obtained by projecting an RL axis as the one axis onto the cross-sectional image.

11. The magnetic resonance imaging apparatus (10) according to claim 10,
wherein the processor (202) is configured to:
in a case where the cross-section (CS) does not include the component in the RL direction, set the horizontal direction and the vertical direction of the cross-sectional image to be the same as a horizontal direction and a vertical direction of a sagittal cross-sectional image.

12. The magnetic resonance imaging apparatus (10) according to any one of claims 1 to 7,
wherein the cross-section (CS) is a reference cross-section from which a two-chamber long-axis image is obtained, and
the processor (202) is configured to:
in a case where the cross-section (CS) includes a component in an HF direction which is a head-foot direction of the subject (130), rotate the cross-sectional image (F9A, F10A) at an angle at which the vertical direction of the cross-sectional image (F9A, F10A) is parallel to a straight line obtained by projecting an HF axis as the one axis onto the cross-sectional image (F9A, F10A).

13. The magnetic resonance imaging apparatus (10) according to claim 12,
wherein the processor (202) is configured to:
in a case where the cross-section (CS) does not include the component in the HF direction, set the horizontal direction and the vertical direction of the cross-sectional image (F9A, F10A) to be the same as a horizontal direction and a vertical direction of an axial cross-sectional image.

14. A positioning assistance method of assisting in setting an imaging position by a magnetic resonance imaging apparatus, the method comprising:
causing a processor (202) to execute:
a step of acquiring a three-dimensional image including a heart of a subject (130) through scanogram imaging executed prior to main imaging;
a step of detecting a landmark of the heart from the three-dimensional image;
a step of setting a position of a cross-section (CS) based on the landmark;
a step of generating a cross-sectional image (F9A, F10A) appearing on the cross-section from the three-dimensional image;
a step of setting a horizontal direction or a vertical direction of the cross-sectional image (F9A, F10A) such that the horizontal direction or the vertical direction of the cross-sectional image (F9A, F10A) is parallel to a straight line obtained by projecting one axis selected from among three axes that form a three-dimensional orthogonal coordinate system of a real space onto the cross-sectional image (F9A, F10A); and
a step of storing the cross-sectional image (F9A, F10A) in a memory (204).

15. A non-transitory, computer-readable recording medium which records thereon, a program for causing a computer to execute the positioning assistance method according to claim 14.
